# EUROPEAN PATENT SPECIFICATION

(11) **EP 3 922 313 B1**
(45) Date of publication and mention of the grant of the patent: **20.05.2026**
(21) Application number: 20749091.3
(22) Date of filing: 31.01.2020
(51) Int. Cl.: A61K 36/062, A61P 35/00, A61P 37/04, A61P 17/16, A61Q 19/00, A61K 8/9728, C12N 1/16, A61Q 19/08, A61K 8/14, C12R 1/645

(54) **EXOSOMES FROM YEAST MASH, MOROMI OF SAKE AND AGED MISO FOR TREATING CANCER AND INFECTIONS AND COSMETIC USES**
EXOSOME AUS HEFE-MAISCHE, MOROMI VON SAKE UND GEREIFTEM MISO ZUR BEHANDLUNG VON KREBS UND INFEKTIONEN SOWIE FÜR KOSMETISCHE ANWENDUNGEN
EXOSOMES ISSUS DE MOÛT DE LEVURE, DE MOROMI DE SAKÉ ET DE MISO VIEILLI POUR LE TRAITEMENT DU CANCER ET DES INFECTIONS ET POUR DES UTILISATIONS COSMÉTIQUES

(30) Priority: 01.02.2019 JP 2019017296
(43) Date of publication of application: 15.12.2021
(73) Proprietor: Da Vinci Universale Co., Ltd., Tokyo, 100-0005 (JP); DASSAI Inc., Iwakuni-shi, Yamaguchi, 742-0422 (JP)
(72) Inventor: MURANAKA, Asao, Kashiwa-Shi, Chiba 277-0832 (JP)
(74) Representative: J A Kemp LLP
(86) International application number: PCT/JP2020/003734
(87) International publication number: WO 2020/158930

(56) References cited:
- WO-A1-2019/088656
- WO-A1-2019/212293
- WO-A1-2020/050521
- CN-A- 106 722 209
- JP-A- 2009 029 788
- JP-A- 2015 119 697
- KR-A- 20110 082 481
- KR-A- 20180 003 344
- WATANABE MITSUHIRO: "The splendor of Japaneses food", JOURNAL OF JAPANESE SOY SAUCE RESEARCH INSTITUTE, vol. 42, no. 2, 2016, pages 123 - 133, XP009530075
- MINETOKI TOSHITAKA: "A characteristic and inflection of the enzyme processing sake lees seasoning", A TECHNICAL JOURNAL ON FOOD CHEMISTRY & CHEMICALS, vol. 2013, no. 1, 1 January 2013 (2013-01-01), JP, pages 39 - 46, XP009530079, ISSN: 0911-2286
- YAMASAKI-YASHIKI SHINO, MIYOSHI YUKI, NAKAYAMA TOMOYA, KUNISAWA JUN, KATAKURA YOSHIO: "IgA-enhancing effects of membrane vesicles derived from Lactobacillus sakei subsp. sakei NBRC15893", BIOSCIENCE OF MICROBIOTA, FOOD AND HEALTH, vol. 38, no. 1, 1 November 2018 (2018-11-01), pages 23 - 29, XP055726840

## Description

### TECHNICAL FIELD

The present invention relates to medical and cosmetic uses of compositions using a yeast-derived exosome.

### BACKGROUND ART

Probiotic lactic acid bacteria have been reported to have a preventive effect on metabolic syndrome such as an effect of inhibiting fat accumulation and an effect of improving insulin resistance. As its mechanism, it is considered that exosomes released from orally ingested lactic acid bacteria and absorbed from an intestinal tract interact with tissues, such as adipose tissue and skeletal muscle, locating far from the intestinal tract, and contribute to prevention of metabolic syndrome. Such effects are highly likely to be provided by exosomes released from many bacteria and yeasts which are widely known to be good for human bodies, and also from many vegetables and fruits which are ingested orally, and their usefulness is highly

KR 2011 0082481 discloses fermented food-derived (e.g. miso) extracellular vesicle for preventing or treating inflammatory diseases and cancer. WO2019088656 discloses yeast-derived extracellular vesicles for healing wound, treating scar, improving wrinkles, skin elasticity, and infections.

### SUMMARY OF THE INVENTION

### PROBLEMS TO BE SOLVED BY THE INVENTION

Since yeast mash, row material for producing sake, is obtained by pure mass culture of yeast, the inventors have considered that the yeast mash could be a rich reservoir of exosomes, and for the first time, have focused on an exosome present in a culture solution obtained from the yeast mash that is used as row material in the production process of sake. In addition, for the first time, the inventors have focused on an exosome contained in miso that contains yeast similarly to sake, particularly, miso that has been aged for one year or more. Therefore, the present invention provides compositions for supressing cancer, for preventing loss of tissue elasticity, and for improving elasticity, containing a novel active ingredient.

### MEANS FOR SOLVING THE PROBLEMS

As a result of intensive studies on the purification process, the inventors have found that an exosome can be extracted by a unique production method. The inventors have examined a change in intracellular granzyme amount in immune cells, and found that the amount of intracellular granzyme increases by addition of exosomes purified from yeast mash and moromi. There was no change in cytokine secretion observed, and no difference in beta-galactosidase staining which is an index of aging in fibroblasts is observed. As another index of aging, expression of collagenase and expression of elastin in aged fibroblasts were evaluated. As a result, it was confirmed that expression levels of collagen and elastin were increased. Accordingly, the inventors have found for the first time that exosomes derived from yeast mash and moromi of sake exert preventing effect on loss of tissue elasticity and improving effect in elasticity.

Furthermore, the inventors have examined changes in gene amounts of collagen type I and elastin, which are known to have an anti-aging effect, in aged fibroblasts, and found that the gene amounts increase depending on the addition of exosomes obtained from miso. In addition, the inventors have found that an immune activity of NK cells is enhanced by adding the exosomes collected from miso.

### ADVANTAGEOUS EFFECTS OF THE INVENTION

The present invention is based on the success in extracting valuable components for activation of immune system, anti-aging, and body health maintainance. The active ingredient containing an exosome obtained by this unique production method can be applied to products for health maintenance in all forms such as sake and miso as well as other beverages, foods, cosmetics, and pharmaceuticals.

### BRIEF DESCRIPTION OF THE DRAWINGS

FIG. 1 is a photograph showing a result of centrifuging yeast mash and moromi of Dassai at 15,000×g at 4°C for 15 minutes. The supernatant was used for exosome extraction.
FIG. 2 shows graphs indicating results of measuring the number of particles of the obtained exosome fractions derived from yeast mash (A) and moromi (B) by a nanoparticle analysis system NanoSight (analysis condition: camera level 14, detection threshold 7). A peak of the particle diameter was observed at 95 nm in the exosomes derived from the yeast mash and at 109 nm in the exosomes derived from the moromi.
FIG. 3 shows graphs indicating results of evaluating cell proliferation inhibition and apoptosis after adding each exosome at concentrations of 0.5, 1, and 2 µg/mL to HCT116 cells. For apoptosis, a measurement result of caspase activity is indicated. Further,
FIG. 4 shows graphs indicating results of proliferation inhibition tests for a colorectal cancer cell line HCT116, a lung cancer cell line A549, a breast cancer cell line MDA-MB-231, and a prostate cancer cell line PC-3M. A vertical axis represents a relative value when the value of control (without addition of exosome) is 1.
FIG. 5 shows graphs indicating results of apoptosis inducibility tests for the colorectal cancer cell line HCT116, the lung cancer cell line A549, the breast cancer cell line MDA-MB-231, and the prostate cancer cell line PC-3M). A vertical axis represents a relative value when the value of control (without addition of exosome) is 1.
FIG. 6 shows photographs of results of a migration property test. A linearly white portion at the center is a scratched portion.
FIG. 7 shows a graph indicating results of the migration property test shown in FIG. 6. A vertical axis represents a relative value when the value of control (PBS) is 1.
FIG. 8 shows expression levels of granzyme in NK cells when exosome is added. The upper figure shows photographs of Western blotting showing expression levels of each protein. The lower figure shows a graph that quantifies expression levels by Western blotting.
FIG. 9 shows graphs indicating results of evaluating an action of exosome by NK cells on secretion amounts of three types of cytokines: interferon gamma, interleukin 17 and TNF alpha.
FIG. 10 shows photographs of results of performing beta-galactosidase staining in order to measure an aging retarding ability of exosomes. When cells are stained green, it indicates that the cells are aged.
FIG. 11 is a graph indicating results of evaluating an action of exosomes on expression levels of collagen type I and collagen type III which are important as subcutaneous collagen, and elastin. Bar graphs shown in each group (control (not added), EtOH, 3 µl of moromi, 10 µl of moromi, 30 µl of moromi, 3 µl of yeast mash, 10 µl of yeast mash, and 30 µl of yeast mash, in order from left) indicate results of collagen type I α1 chain, collagen type I α2 chain, collagen type III, and elastin, in order from left. A vertical axis represents a relative value when the value of control is 1.
FIG. 12 shows expression levels of granzyme in NK cells when exosomes are added (n=3). The expression levels by Western blotting were quantified and expressed as average values.
FIG. 13 is a graph indicating results of evaluating an action of exosomes on expression levels of collagen type I and elastin. Bar graphs shown in each group (non-aged, one-year aged, quick brewed, and three-year aged in order from left) indicate results of collagen type I on the left and elastin on the right. A vertical axis represents a relative value when the value of control is 1.

### MODE FOR CARRYING OUT THE INVENTION

### 1. Preparation of Exosomes Derived from Yeast Mash and Moromi or Miso

Yeast mash is obtained by adding yeast to a mixture of steamed rice, malted rice, and water and culturing, for brewing sake. Fermentation of yeast of the yeast mash proceeds, and a mixture obtained by adding steamed rice, malted rice, and water thereto in several times is moromi. Both yeast mash and moromi are usually used in the industry of sake production, and the production method thereof is also widely known. In particular, yeast mash and moromi used for Dassai are available from Asahi Shuzo Co., Ltd. (2167-4, Shutomachi Osogoe, Iwakuni-shi, Yamaguchi). Extraction of exosomes from yeast mash and moromi can be performed by, for example, (a) centrifuging yeast mash or moromi of sake; (b) ultracentrifuging the supernatant obtained in (a) above; and (c) collecting the precipitate obtained in (b) above as an exosome fraction.

Miso is obtained by mixing malted rice and salt to soybean and fermenting the mixture. "Non-aged miso" refers to miso that has not been subjected to aging processing, and "aged miso" refers to miso that has been produced by natural brewing, aged by allowing to stand using power of natural fermentation of natural yeasts and lactic acid bacteria. The aged miso is called one-year aged miso, three-year aged miso, or the like, depending on the aging period. "Fast-aged miso" refers to miso made in a short period of time by artificially being placed at a certain temperature and subjected to heat treatment, so as to activate action of malted rice (koji) to forcibly ferment miso, and examples thereof include fast-aged miso that has been aged in one month, and the like. The miso in the present specification is preferably aged miso, and examples thereof include miso that has been aged for one to three years, but the miso is preferably aged for one year or more, and more preferably aged for three years or more. Extraction of exosomes from miso can be performed by, for example, (a) centrifuging miso or a suspension of miso (for example, one obtained by dissolving miso in water); (b) ultracentrifuging the supernatant obtained in (a) above; and (c) collecting the precipitate obtained in (b) above as an exosome fraction.

The centrifugation in step (a) can be performed at 10,000 to 20,000×g, 10,000 to 15,000×g, 12,000 to 17,000×g, or 15,000×g. Further, the centrifugation in step (a) can be performed, for example, for 5 minutes or more, 10 minutes or more, or 15 minutes or more. Furthermore, the centrifugation in step (a) can be performed at 4°C to room temperature. The supernatant obtained in step (a) is used for recovery of exosomes. The supernatant can be subjected to filter filtration (for example, filtration with a 0.65 µm filter) as necessary to further remove residue. The filter filtration can be performed in two stages as necessary.

The ultracentrifugation in step (b) can be performed at 70,000 to 100,000×g, 10,000 to 210,000×g, 50,000 to 200,000×g, or 100,000 to 210,000×g. The ultracentrifugation in step (b) can be performed, for example, for 50 minutes or more, 60 minutes or more, or 70 minutes or more, for example, for 60 minutes to 4 hours. In addition, the ultracentrifugation in step (b) can be performed at 4°C to room temperature. By collecting the precipitate obtained in step (b) as exosomes, exosomes derived from yeast mash or moromi can be obtained (step C). The recovered exosomes can be washed with PBS or the like as necessary. The washing can be performed by suspending the precipitate in PBS and then centrifuging at 100,000 to 210,000×g to remove the supernatant. The conditions for centrifugation can be set according to the centrifugation in step (b).

Whether or not an exosome is contained in the obtained exosome fraction can be confirmed, for example, by measuring the number of particles using nanoparticle analysis system NanoSight in accordance with the description of examples of the present application, or measuring the amount of contained protein.

Alternatively, it is also possible to acquire a fraction containing an exosome as a main component by using other methods, and an exosome separated and concentrated under such other conditions is also included in the exosome of the present invention. For example, it can also be extracted using a Total Exosome Isolation reagent (Thermo Fisher Scientific) or the like. Ultrafiltration, pelleting.

The exosome used in the present invention is preferably an exosome obtained by the above-described method. The exosome is a membrane endoplasmic reticulum with a diameter of 30 to 200 nm surrounded by a lipid bilayer membrane secreted from a cell, and usually contains nucleic acids such as mRNA and miRNA, proteins, and the like. The average particle diameter of the exosome is preferably 80 to 200 nm, 100 to 180 nm, 110 to 160 nm, or 118 nm. In addition, the peak diameter of the exosome can be preferably 70 to 140 nm, 80 to 130 nm, 90 to 120 nm, or 95 nm.

### 2. Composition Containing Exosome

Compositions containing the exosome are also disclosed. The content of the exosome in the composition is not particularly limited as long as it is a concentration that can achieve the intended use of the composition, and can be set to, for example, 1% by mass or more, 2% by mass or more, 3% by mass or more, 4% by mass or more, 5% by mass or more, 10% by mass or more, 20% by mass or more, 30% by mass or more, 40% by mass or more, or 50% by mass or more. In addition, the amount of the exosome contained in the composition can be set to 4×10⁸/ml or more, 5×10⁸/ml or more, 1×10⁹/ml or more, 1×10¹⁰/ml or more, 1×10¹¹/ml or more, or 1×10¹²/ml or more. Alternatively, the amount of the exosome contained in the composition can be set to 33 ng/ml or more, 50 ng/ml or more, 100 ng/ml or more, 500 ng/ml or more, 1 µg/ml or more, 5 µg/ml or more, 10 µg/ml or more, 500 µg/ml or more, 1 mg/ml or more, 5 mg/ml or more, 10 mg/ml or more, 50 mg/ml or more, 100 mg/ml or more, 500 mg/ml or more, 1 g/ml or more, or 5 g/ml or more as the amount of protein contained in the exosome.

Components other than the exosome in the composition can be appropriately selected depending on the purpose of use, and can contain, for example, food, a food additive, a cosmetic raw material, a cosmetic additive, a pharmaceutical additive, and the like. Such an exosome composition can be, for example, to produce food (including health food or a nutrient), a cosmetic, or a pharmaceutical, which can be produced by appropriately adding as raw materials for these in the production process.

The shape of the composition is not particularly limited, and can be appropriately selected depending on a use form. Examples thereof include a powder form, a particle form, a granular form, a tablet form, a rod form, a plate form, a liquid form, a candy form, a paste form, a cream form, a capsule form such as a hard capsule or a soft capsule, a caplet form, a tablet-like form, a gel form, a jelly form, a cream form, and the like.

Since the exosome used in the present invention has a cancer cell proliferation inhibition effect, the composition containing the exosome can be a composition for inhibiting cancer cell proliferation. In the present specification, the "cancer " includes epithelial malignancies, hematopoietic malignancies derived from spinal cord, and the like, and specifically includes hematopoietic cell malignancies such as lymphoma (Hodgkin's disease, non-Hodgkin lymphoma, and the like) and multiple myeloma; ovarian cancer; breast cancer; mammary gland cancer; uterine cancer such as uterine corpus cancer and cervical cancer; endometrial cancer; ovarian cancer such as non-mucinous ovarian cancer; esophageal cancer; stomach cancer; appendix cancer; colorectal cancer such as colorectal cancer, rectal cancer, and colon cancer; liver cancer such as hepatocellular cancer; gallbladder cancer; bile duct cancer; pancreatic cancer; adrenal cancer; gastrointestinal stromal tumor; mesothelioma such as pleural mesothelioma; head and neck cancer such as laryngeal cancer, throat cancer, and oral cancer (oral floor cancer, gingival cancer, tongue cancer, buccal mucosa cancer, and the like); brain cancer such as ependymoma; salivary gland cancer; sinus cancer (maxillary sinus cancer, frontal sinus cancer, ethmoid sinus cancer, sphenoid sinus cancer, and the like); thyroid cancer; lung cancer; lung adenocarcinoma; osteosarcoma; prostate cancer; testicular tumor or testicular cancer, such as testicular choriocarcinoma; kidney cancer such as renal cell cancer; bladder cancer; sarcomas such as rhabdomyosarcoma; skin cancer; anal cancer; or cancer of the blood such as chronic myelogenous leukemia and acute myelogenous leukemia, or metastatic cancer thereof. Preferably, the cancer is colorectal cancer, prostate cancer, or breast cancer.

In addition, since the exosome has an action of promoting granzyme expression by NK cells, the composition containing the exosome can be used for promoting granzyme expression by NK cells. Granzyme is a serine protease that plays a central role in cytotoxic activity by NK cells. Therefore, the composition for promoting granzyme expression by NK cells can be used as a pharmaceutical composition for treating or preventing cancer using an ability of NK cells to damage cancer cells, and can also be used as a composition for treating viral or bacterial infection or inflammatory disease.

Furthermore, the exosome was confirmed to have a collagen and/or elastin production promoting action. Therefore, the composition containing the exosome can be used for promoting production of collagen and/or elastin. Fiber components such as collagen and elastin are known to play an important role in elasticity of tissues such as skin and blood vessels. Therefore, the composition for promoting production of collagen and/or elastin can be used as a composition for preventing loss of tissue elasticity or improving tissue elasticity. In particular, when the tissue is skin, the composition can also be used for cosmetic. More specifically, it can be used for improving appearance of aged skin, and reducing sagging and/or wrinkles or small wrinkles. The cosmetic composition may be applied to the skin or may be orally ingested into the body.

A use amount or administration amount, and a use method or administration method of the composition can be appropriately set depending on the form or purpose of the composition. For example, when ingested as food, an adult can ingest 0.01 mg to 100 g at one time, several times per day.

### EXAMPLES

Hereinafter, the present invention will be specifically described with reference to examples of the present invention, but the present invention is not limited thereto.

For the purpose of evaluating effects on cancer cells, immune cells, and senescent cells, the following experiment was performed on exosomes recovered from yeast mash and moromi used for Dassai of Asahi Shuzo Co., Ltd.

### (Example 1) Preparation of Exosome Sample for Evaluation

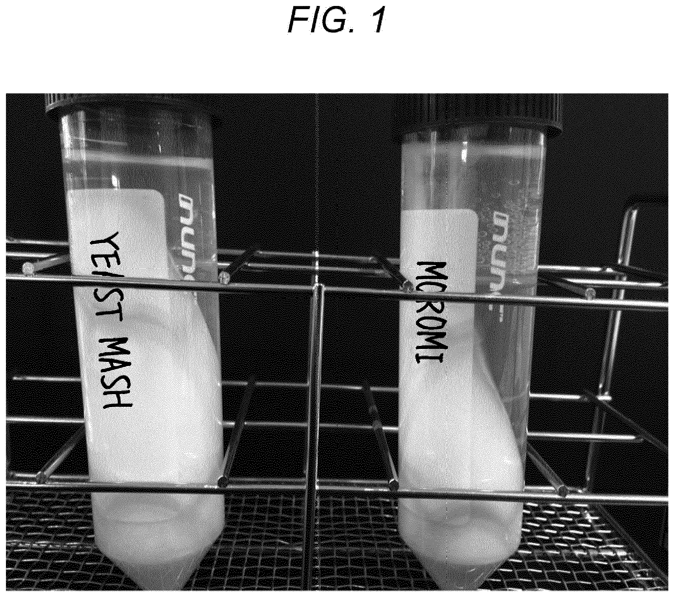

(1) Yeast mash and moromi of Dassai (Asahi Shuzo Co., Ltd.) were each centrifuged at 15,000×g at 4°C for 15 minutes, and the supernatant was filtered through a 0.65 µm filter (FIG. 1). The supernatant after filtration was ultracentrifuged at 35,000 rpm (100,000×g to 210,000×g) at 4°C for 70 minutes, using an ultracentrifuge and a SW41 Ti rotor manufactured by Beckman Coulter, Inc. After centrifugation, the supernatant was discarded, and for washing, the precipitate was suspended in PBS, and then ultracentrifugation was performed again under the same conditions. After washing the sample, an exosome fraction remaining on a bottom surface of a tube was recovered in a low adsorption tube. The amount of supernatant after ultracentrifugation was 88 mL for the yeast mash and 176 mL for the moromi. The precipitates of yeast mash and moromi were finally suspended in 170 µL and 340 µL of PBS, respectively.
(2) As non-aged miso, common miso not subjected to aging processing was used. As one-year to three-year aged miso, miso produced by natural brewing, which was prepared by waiting for natural aging in a tub for one to three years using power of natural fermentation of natural yeasts and lactic acid bacteria without artificial heating, was used. As the fast-aged miso, one that was aged in one month by a quick brewing method for making miso in a short period of time was used, the method including artificially being placed at a certain temperature and subjected to heat treatment, so as to activate action of malted rice (koji) to forcibly ferment miso. These misos were centrifuged at 15,000×g at 4°C for 15 minutes, and the supernatants were each filtered through a 0.65 µm filter (FIG. 1). The supernatant after filtration was ultracentrifuged at 35,000 rpm (100,000×g to 210,000×g) at 4°C for 70 minutes, using an ultracentrifuge and a SW41 Ti rotor manufactured by Beckman Coulter, Inc. After centrifugation, the supernatant was discarded, and for washing, the precipitate was suspended in PBS, and then ultracentrifugation was performed again under the same conditions. After washing the sample, an exosome fraction remaining on a bottom surface of a tube was recovered in a low adsorption tube and used.

### (Example 2) Measurement of Particle Concentration and Protein Concentration of Prepared Exosome

(1) As for exosome recovered from yeast mash and moromi of Dassai, the yeast mash-derived exosome was diluted 100 times with PBS, and the moromi-derived exosome was diluted 40 times with PBS. The number of particles of each diluted solution was measured with a nanoparticle analysis system NanoSight (analysis condition: camera level 14, detection threshold 7). Regarding the protein concentration, protein quantification was performed using 20 µL of an exosome solution, with Qubit Protein Assay Kit (Invitrogen).

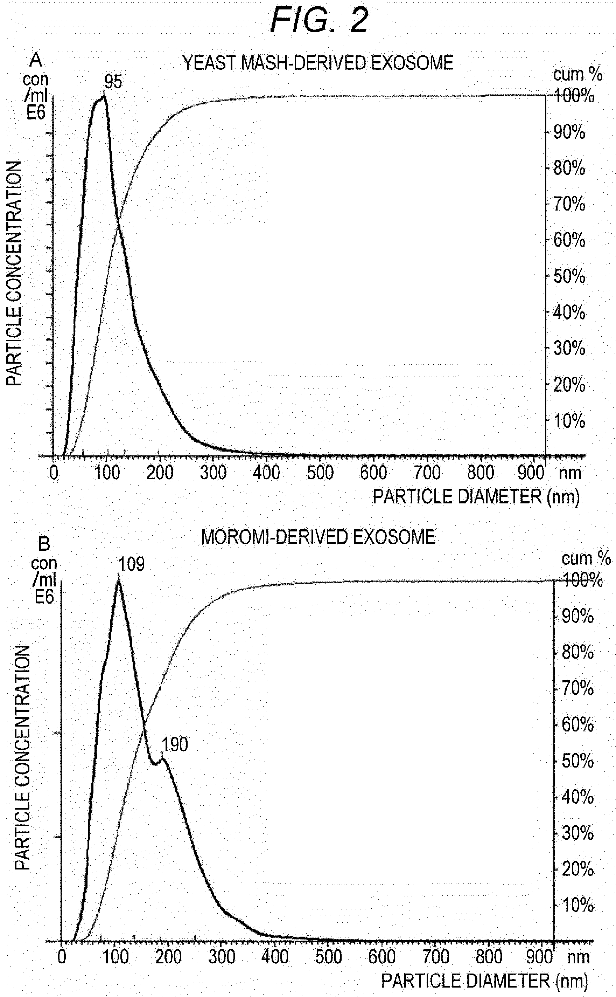

The number of particles and protein concentration of the exosomes recovered from yeast mash and moromi of Dassai using the nanoparticle analysis system are shown in Table 1 below. In addition, FIG. 2 shows a particle size distribution diagram of the particles obtained by the nanoparticle analysis system. The amount of exosomes contained in the yeast mash and the moromi was 3.8×10⁸/ml for the yeast mash and 0.37×10⁸/ml for the moromi. Moreover, the protein concentrations of exosomes contained in the yeast mash and the moromi were 32.1 ng/ml for the yeast mash and 18.1 ng/ml for the moromi.

**[Table 1]**

| | Stock solution concentration (×10⁸) | Average particle diameter (nm) | Peak particle diameter (nm) | Protein concentration (ng/ml) | Number of particles per 1 mL of supernatant (×10⁸/mL) |
|---|---|---|---|---|---|
| Yeast mash-derived exosome | 1708 | 118 | 95 | 32.1 | 3.3 |
| Moromi-derived exosome | 190 | 155 | 109 | 18.1 | 0.37 |

(2) After dissolving 1 g of exosome recovered from each miso in physiological saline, the number of particles was measured by NanoSight (analysis condition: camera level 14, detection threshold 7).

As a result of the measurement, the number of particles was as follows: 1.2×10¹⁰/ml for the non-aged miso, 4.7×10¹¹/ml for the one-year aged miso, 7.8×10¹²/ml for the three-year aged miso, and 2.5×10¹⁰/ml for the fast-aged miso.

(Example 3) Proliferation Test and Cell Death Test in HCT116 Cell HCT116 cells (human colon adenocarcinoma-derived cancer cells) were seeded in a 96-well plate at 2,000 cells/well, and the next day, the exosome prepared in Example 1 was added at concentrations of 0.5, 1, and 2 µg/mL. After 3 days, a cell proliferation test was performed to evaluate cell proliferation.

Cell death by apoptosis was evaluated by measuring caspase activity. The medium was removed from the cells of (3) for which the cell proliferation test had ended, and 100 µl of M-PER Mammalian Protein Extraction Reagent (Thermo Fischer Scientific) was added to lyse the cells to obtain a cell lysate. 50 µl of the cell lysate and 50 µl of a solution of Caspase-Glo (registered trademark) 3/7 Reagent (Caspase-Glo (registered trademark) 3/7 Assay Systems, Promega Corporation) were mixed, and the caspase activity was measured by luminescence.

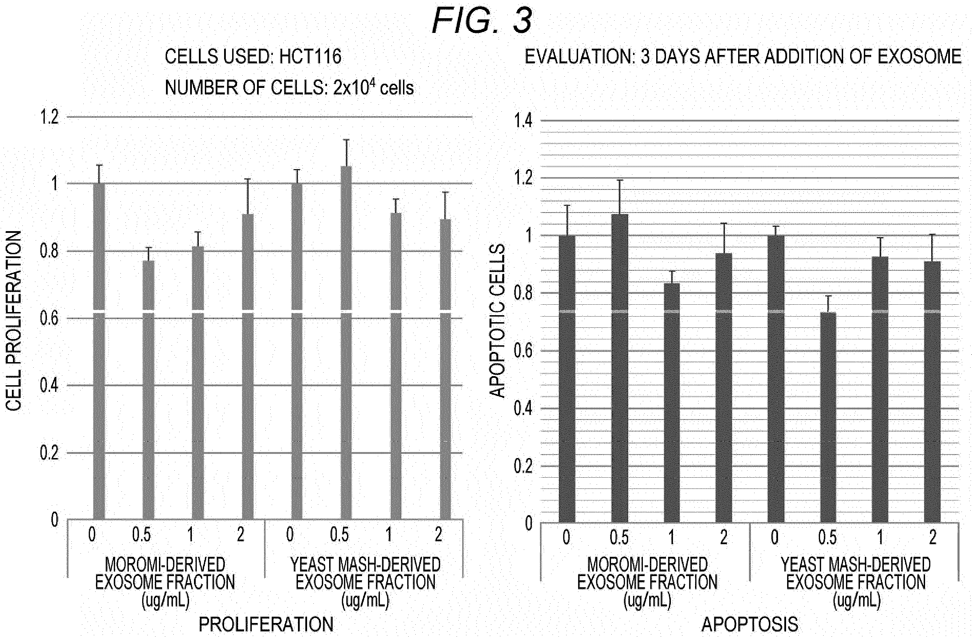

As shown in FIG. 3, when the exosomes derived from the moromi and the yeast mash were added to the colorectal cancer cell line HCT116 at concentrations of 0.5, 1, and 2 µg/mL, proliferation inhibitory activity was observed at 0.5 µg/mL or more for the moromi and at 1 µg/mL or more for the yeast mash, as compared to control (PBS). However, as for apoptosis, no significant difference was observed as compared to the control. Therefore, it was considered that the effect of exosomes derived from the yeast mash and the moromi in the colorectal cancer cell line was cell proliferation inhibitory activity.

### (Example 4) Proliferation Test and Cell Death Test in Various Cancer Cell Types

In order to confirm whether there is a similar effect also in cancer cells other than colon cancer, a proliferation inhibition test and an apoptosis induction ability test were performed for more cancer cell types (colorectal cancer cell line HCT116, lung cancer cell line A549, breast cancer cell line MDA-MB-231, and prostate cancer cell line PC-3M) in the same manner as in Example 3 except that the exosome concentration was set to 2 µg/mL.

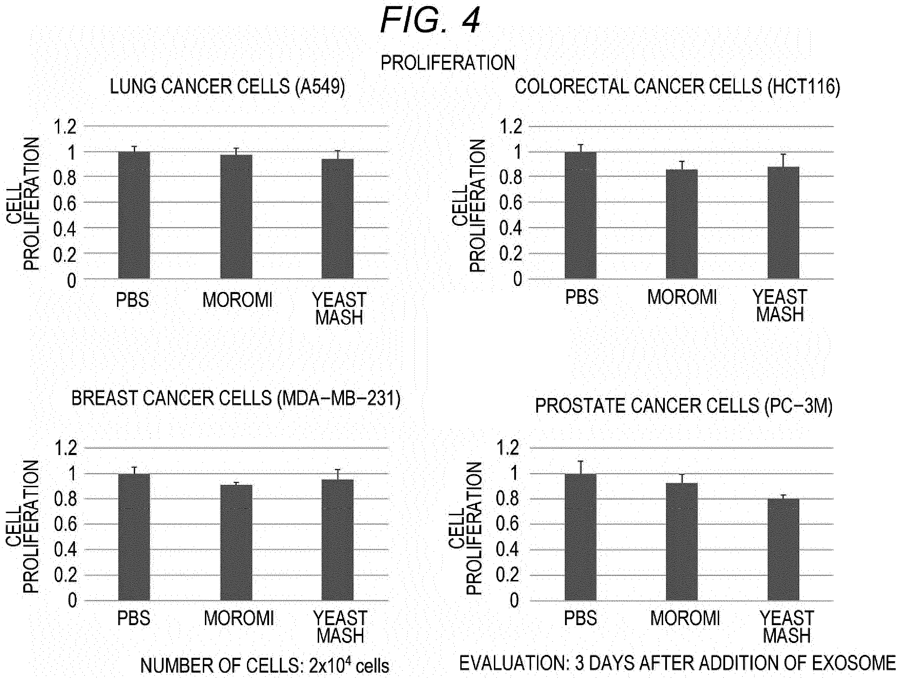

As shown in FIG. 4, a proliferation inhibition effect was reproduced in the colorectal cancer cell line as in Example 2. In addition, an effect equivalent to that of colorectal cancer was also observed in the prostate cancer cell line and the breast cancer cell line, and a proliferation inhibition effect was also observed in lung cancer although being relatively weak.

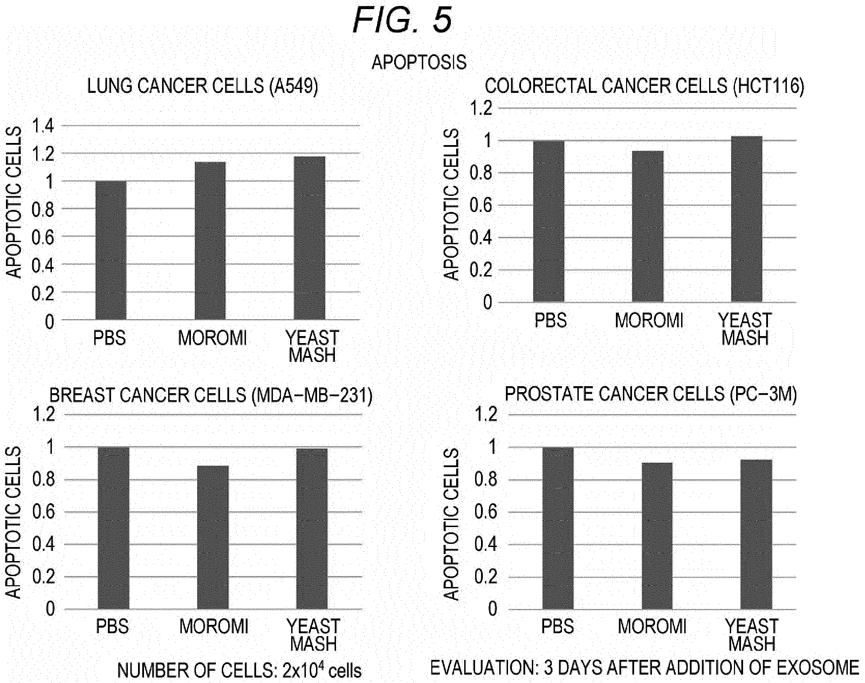

Results of apoptosis measurements are shown in FIG. 5. No significant apoptosis induction was found in all cell lines as a result of evaluation in relative amounts compared to PBS. Therefore, it was reconfirmed that the cell proliferation inhibition effect observed in FIG. 4 was not also due to cell death induction but due to inhibition of cell proliferation.

### (Example 5) Migration Property Test in Cancer Cell

HCT116 cells were seeded in a 24-well plate, and the next day, the inside of the plate was scratched to form a partial space. The cells were photographed on Day 0, Day 1 and Day 2, with the scratched day as Day 0, and how much the scratched part was narrowed by migration of the cells was evaluated.

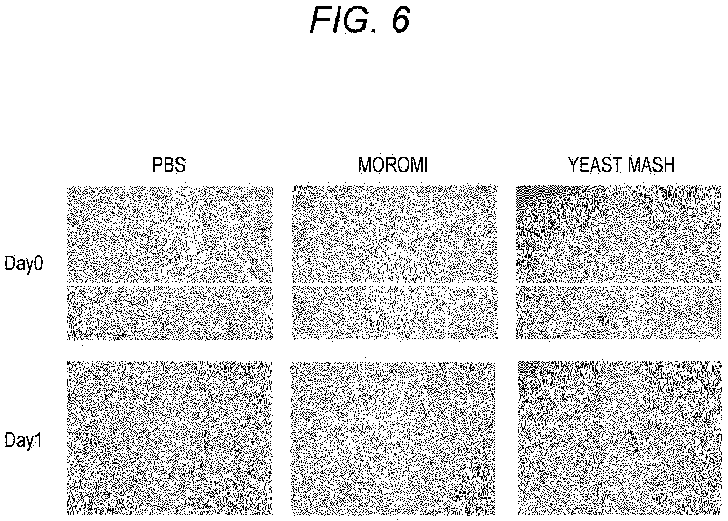

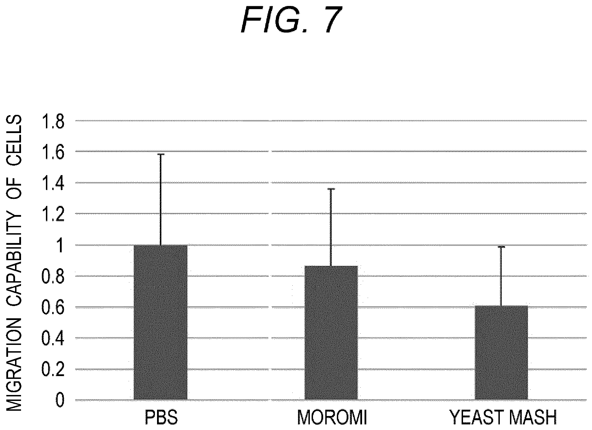

Results of the migration property test are shown in FIG. 6 (photograph) and FIG. 7 (graph). As for the migration ability, a tendency of inhibition with moromi and yeast mash was observed, but a remarkable inhibition effect was not observed.

An exosome fraction was successfully isolated from each of the yeast mash and the moromi. As a result of evaluating proliferation inhibition ability and cell death-inducing ability in cancer cells, a proliferation inhibition effect was observed in the cell lines of prostate cancer, colorectal cancer and breast cancer, and a weak proliferation inhibition effect was observed in the lung cancer cell line. Since it was confirmed that cell death of these cell lines was not induced, it was found that the exosomes derived from the yeast mash and the moromi have an effect of inhibiting only proliferation of cells without causing cancer cells to die. In addition, with respect to the migration ability of cancer cells, a tendency of inhibition was observed, but no remarkable change was observed. Based on the above, an effect of inhibiting proliferation of cancer cells was observed in the exosomes derived from the yeast mash and the moromi, but it was considered that this effect was not so strong as to induce cell death as an anticancer agent, but was an effect of gently stopping the proliferation of cancer cells.

**[Table 2]**

| Effect of Dex | Human cancer cells | | | |
|---|---|---|---|---|
| | Colorectal cancer | Breast cancer | Prostate cancer | Lung cancer |
| Cell proliferation inhibition | +++ | + | +++ | +/- |
| Cytocidal effect | - | - | - | - |
| Infiltration inhibition ability | - | - | - | - |

### (Example 6) Expression Analysis of Granzyme in Immune Cell

(1) NK-92MI cells, which are NK cells that can be cultured for a long time, were seeded in a 24-well plate at 25,0000 cells/well, and the exosomes prepared in Example 1 were added so as to be 3, 10, or 30 µ/L (for the miso exosome, approximately 100 particles per cell, i.e. 2×10⁷ particles per well). After 2 days, the cells were recovered, protein extraction was performed, and the expression level of the protein was measured by Western blotting.

### (2) Results of yeast mash exosome

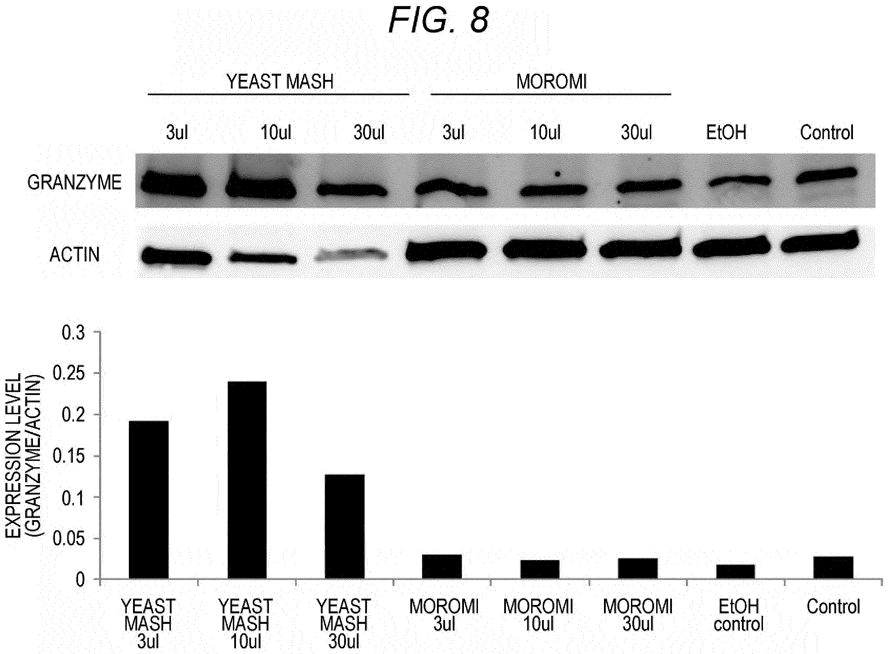

As shown in FIG. 8, an increase in the expression level of granzyme in NK cells was observed by adding the yeast mash exosome. The upper figure of FIG. 8 shows photographs of the Western blotting for evaluating the expression levels of protein, and the lower figure shows a graph that quantifies the expression levels. Based on the above, it is considered that the yeast mash exosome contributes to a cytocidal effect by increasing expression of granzyme in NK cells. Regarding the moromi exosome, no significant change was observed in the expression level of granzyme.

### (3) Results of miso exosome

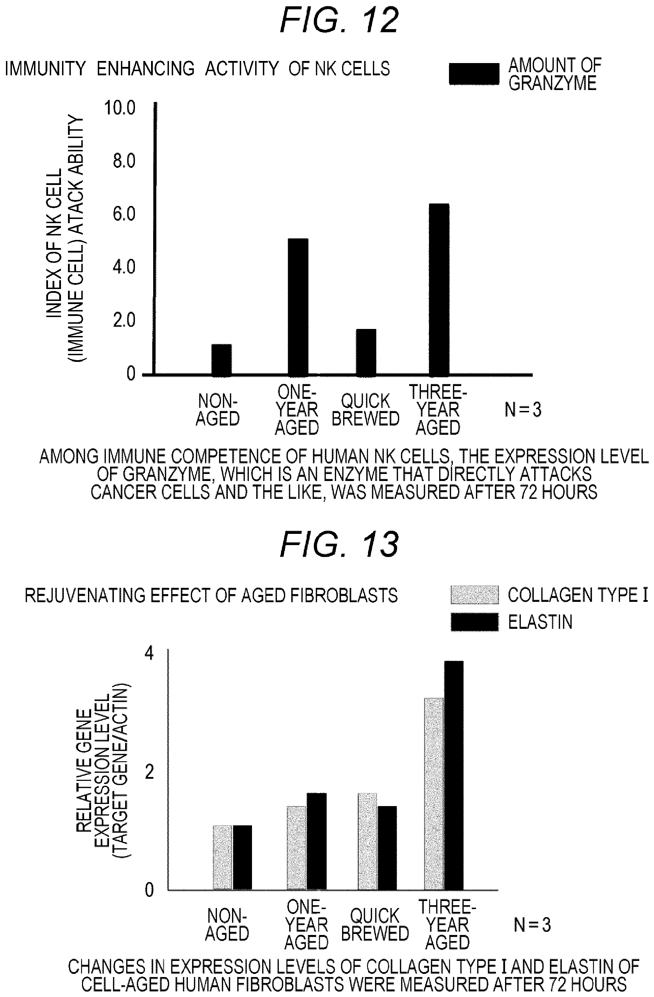

As shown in FIG. 12, an increase in the expression level of granzyme in NK cells was observed by adding the exosomes of one-year and three-year aged miso. Therefore, it is considered that the exosome of miso aged for one year or more contributes to the cytocidal effect by increasing the expression of granzyme in NK cells.

### (Example 7) Measurement of Secretion of Cytokine in Immune Cell

As cytokine secretion of NK cells, secretion of three types of main cytokines: interferon gamma, interleukin 17, and TNF alpha, were evaluated. NK-92MI cells were seeded in a 24-well plate at 25,0000 cells/plate, and the exosomes were added so as to be 3, 10, or 30 µ/L. After 2 days, the culture supernatant was recovered, and centrifuged at 300 g for 3 minutes to recover the supernatant. The measurement of interferon gamma, interleukin 17, and TNF alpha in the supernatant was performed using an ELISA kit (R&D systems).

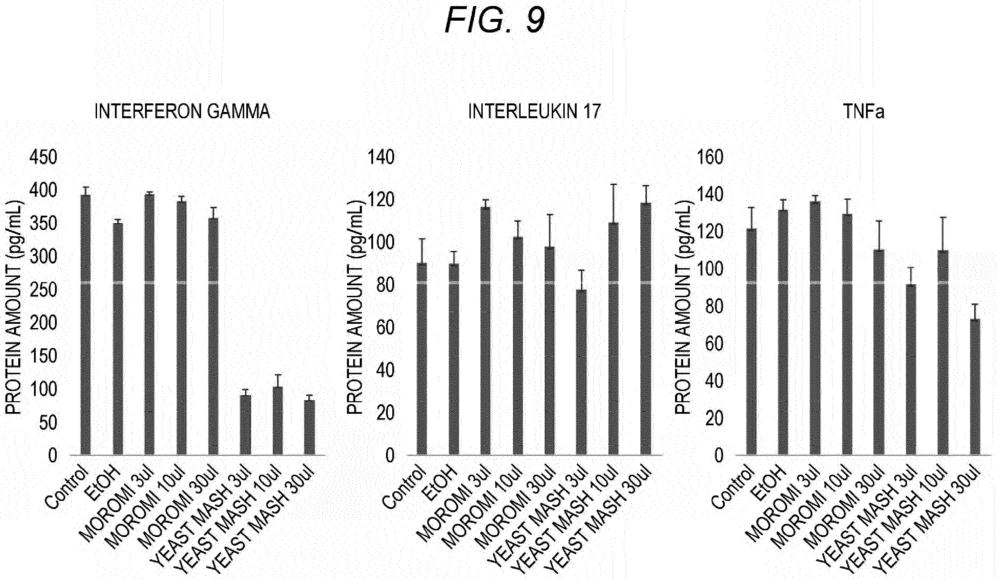

Results are shown in FIG. 9. Although a large increase in the secretion was not observed in any of the cytokines, the secretion of interferon gamma decreased in the yeast mash exosome.

### (Example 8) Beta-Galactosidase Staining in Senescent Cell

Beta-galactosidase staining was performed as a method for evaluating senescent cells. SPiDER-βGal (DOJINDO LABORATORIES), which is known to be highly sensitive, was used for beta-galactosidase staining. Specifically, fibroblasts (P8) to which exosomes were added so as to be 3, 10, or 30 µ/L were seeded in a 60 mm dish at 2×10⁵ cells/dish and passaged twice to age the cells. The exosomes were added so as to be 3, 10, or 30 µ/L each time the cells were passaged. Thereafter, aged fibroblasts (P10) were seeded in a 35 mm glass base dish. On the next day, the medium was removed by suction, and the dish was washed once with 2 ml of HBSS. After 72 hours from the start of the culture, 2 ml of a 4% paraformaldehyde/PBS solution was added to immobilize the cells at room temperature for 3 minutes. 2 ml of the 4% paraformaldehyde/PBS solution was removed by suction, and washing was performed twice with 2 ml of HBSS. 2 ml of SPiDER-βGal working solution (DOJINDO LABORATORIES) was added, and the dish was allowed to stand at 37°C for 30 minutes. Thereafter, washing was performed twice with 2 ml of HBSS, and observation was performed with a confocal laser microscope.

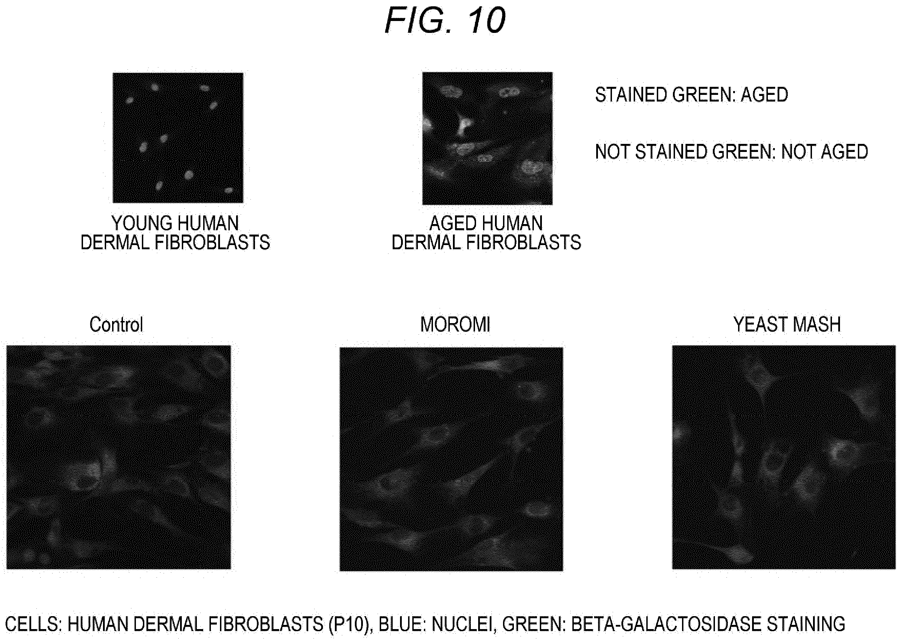

Results are shown in FIG. 10. Since the cells were stained green in both the control and the exosome-added cells, it was shown that the exosomes derived from the moromi and the yeast mash could not stop aging of the cells.

### (Example 9) Measurement of Expression Levels of Collagenase and Elastin in Senescent Cell

(1) Changes in the expression levels of collagen and elastin with reduced activity in aged fibroblasts due to exosome addition were evaluated. Aged fibroblasts (P10) were seeded in a 24-well plate at 100,000 cells/well, and the next day, the exosomes were added so as to be 3, 10, or 30 µ/L (for the miso exosome, approximately 100 particles per cell, i.e. 2×10⁷ particles per well). After 2 days, RNA extraction was performed using Qiagen miRNeasy mini kit. Using the extracted RNA as a sample, RT-PCR was performed using High-Capacity cDNA Reverse Transcription Kit (Applied Biosystems). Taqman Universal Master Mix II (of Applied Biosystems) was used for quantitative PCR. As primers/probes for detection of collagenase and elastin, evaluation was performed using Taqman method of Applied Biosystems. Primers/probes of collagen type I alpha 1 chain, collagen type I alpha 2 chain, collagen type III, and elastin were used.

### (2) Results of yeast mash and moromi exosomes

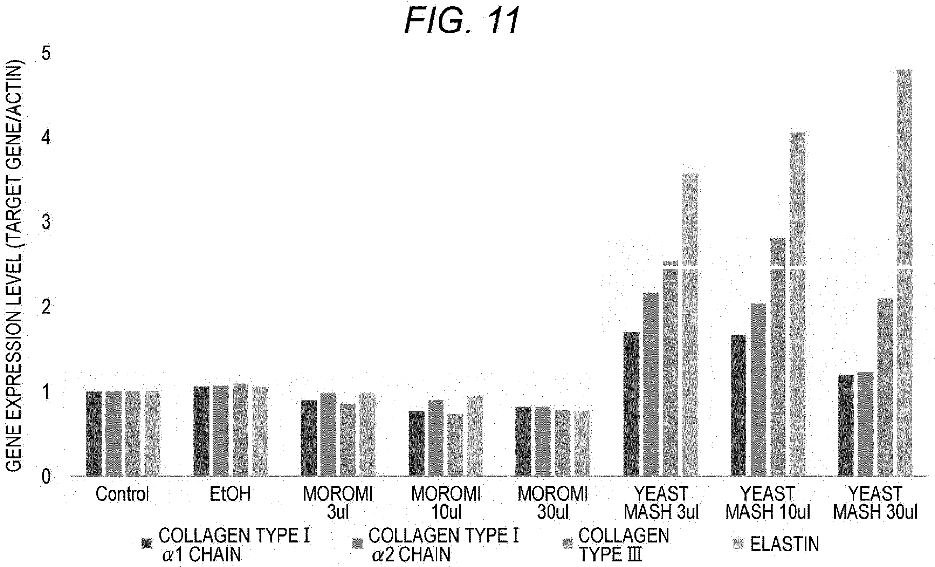

The expression levels of collagen I and collagen III, which are important as subcutaneous collagen, and elastin, were evaluated. Results are shown in FIG. 11. It was found that the expression levels of collagen type I, collagen type III, and elastin were all increased by adding the yeast mash exosome. On the other hand, when the moromi exosome was added, no change was observed in the expression levels of these substances.

### (3) Results of miso exosome

The expression levels of collagen type I and elastin were evaluated. Results are shown in FIG. 13. It was found that the expression levels of collagen type I and elastin were all increased by adding the miso exosome. In particular, when the three-year aged exosome was added, the expression levels of these substances remarkably increased.

As described above, the exosome fractions were isolated from the yeast mash and the moromi as well as the miso, and the functions in immune cells and senescent cells were evaluated. Regarding immune cells, attention was paid to NK cells having a cytocidal effect on cancer cells. As a result, it was found that the amount of granzyme, which is known to have a cytocidal effect, in the cell increased by the addition of the yeast mash exosome. This result suggests that the NK cells more effectively kill many cancer cells when attacking cancer cells. On the other hand, no significant change was observed in the secretion of cytokines that attract other immune cells. Although the secretion of interferon gamma in the yeast mash exosome is reduced, it can be considered that it is a stage in which the NK cells enhance a cytocidal activity, also from the fact that the secretion is inversely correlated with the expression level of granzyme

On the other hand, with respect to the evaluation of aging in the fibroblasts, it was not possible to stop the aging of fibroblasts in both of the yeast mash exosome and the moromi exosome. On the other hand, since production of collagen and production of elastin in aged fibroblasts were enhanced by the addition of the yeast mash exosome, it was suggested that the yeast mash exosome has an action of enhancing the production of collagen and elastin which are reduced by aging of the skin.

Based on the above, it was found that the exosomes derived from yeast (yeast mash, moromi, and miso) enhance the cytocidal effect of NK cells by increasing the expression level of granzyme. In addition, it is considered that for senescent cells, the exosomes can contribute to prevention of decrease in elasticity and improvement in elasticity in the skin by increasing the expression levels of collagen and elastin.

**[Table 3]**

| | Moromi | Yeast mash |
|---|---|---|
| Anti-cancer action | + | + |
| Immunity enhancement effect | - | ++ |
| Anti-aging action | - | - |
| Skin-beautifying effect | - | +++ |

## Claims

1. A composition for use in a method of treating or preventing cancer, the composition comprising an exosome as an active ingredient, the exosome extracted from yeast contained in yeast mash, moromi of sake, miso aged for one or more years, or miso aged for three or more years.

2. The composition for use according to claim 1, comprising the exosome at 4×10⁸/ml or more, or at 33 ng/ml or more, preferably at 4.7×10¹¹/ml or more, more preferably 7.8×10¹²/ml or more as a protein amount.

3. The composition for use according to claim 1 or 2, wherein the cancer is colorectal cancer, prostate cancer, or breast cancer.

4. A composition for use in a method of treating a viral or bacterial infection, the composition comprising an exosome as an active ingredient, the exosome extracted from yeast contained in yeast mash of sake, miso aged for one or more years, or miso aged for three or more years.

5. A cosmetic method of preventing loss of tissue elasticity or for improving tissue elasticity, said method comprising administering a composition comprising an exosome as active ingredient, the exosome extracted from yeast contained in yeast mash of sake, miso aged for one or more years, or miso aged for three or more years.

6. The cosmetic method according to claim 5, wherein the tissue is skin.

7. The composition for use according to any one of claims 1 to 4, or the cosmetic method according to claim 5 or 6, wherein the exosome is produced by a method comprising:
(a) centrifuging the yeast mash or the moromi of sake, or miso aged for one or more years, or miso aged for three or more years at 10,000 to 20,000×g;
(b) ultracentrifuging the supernatant obtained in (a) at 10,000 to 210,000×g; and
(c) collecting a precipitate as an exosome fraction.

## Patentansprüche

1. Zusammensetzung zur Verwendung in einem Verfahren zur Behandlung oder Vorbeugung von Krebs, wobei die Zusammensetzung ein Exosom als Wirkstoff umfasst, wobei das Exosom aus Hefe extrahiert ist, die in Hefebrei, Moromi von Sake, ein oder mehrere Jahre gereiftem Miso oder drei oder mehr Jahre gereiftem Miso enthalten ist.

2. Zusammensetzung zur Verwendung nach Anspruch 1, die das Exosom in einer Menge von 4 × 10⁸/ml oder mehr, oder von 33 ng/ml oder mehr, vorzugsweise von 4,7 × 10¹¹/ml oder mehr, noch bevorzugter von 7,8 × 10¹²/ml oder mehr, umfasst.

3. Zusammensetzung zur Verwendung nach Anspruch 1 oder 2, wobei es sich bei dem Krebs um Darmkrebs, Prostatakrebs oder Brustkrebs handelt.

4. Zusammensetzung zur Verwendung in einem Verfahren zur Behandlung einer viralen oder bakteriellen Infektion, wobei die Zusammensetzung ein Exosom als Wirkstoff umfasst, wobei das Exosom aus Hefe extrahiert ist, die in Hefebrei von Sake, ein oder mehrere Jahre gereiftem Miso oder drei oder mehr Jahre gereiftem Miso enthalten ist.

5. Kosmetisches Verfahren zur Vorbeugung gegen den Verlust der Gewebeelastizität oder zur Verbesserung der Gewebeelastizität, wobei das Verfahren die Verabreichung einer Zusammensetzung umfasst, die ein Exosom als Wirkstoff enthält, wobei das Exosom aus Hefe extrahiert wurde, die in Hefebrei von Sake, ein oder mehrere Jahre gereiftem Miso oder drei oder mehr Jahre gereiftem Miso enthalten ist.

6. Kosmetisches Verfahren nach Anspruch 5, wobei das Gewebe Haut ist.

7. Zusammensetzung zur Verwendung nach einem der Ansprüche 1 bis 4 oder dem kosmetischen Verfahren nach Anspruch 5 oder 6, wobei das Exosom durch ein Verfahren hergestellt wird, das umfasst:
(a) Zentrifugieren des Hefebreis oder des Moromi von Sake oder von ein oder mehrere Jahre gereiftem Miso oder von drei oder mehr Jahre gereiftem Miso bei 10.000 bis 20.000 xg;
(b) Ultrazentrifugieren des in (a) erhaltenen Überstands bei 10.000 bis 210.000 x g; und
(c) Gewinnen eines Niederschlags als Exosomenfraktion.

## Revendications

1. Composition destinée à être utilisée dans un procédé de traitement ou de prévention du cancer, la composition comprenant un exosome en tant que principe actif, l'exosome étant extrait de levures contenues dans du moût de levure, du moromi de saké, du miso vieilli un an ou plus ou du miso vieilli trois ans ou plus.

2. Composition destinée à être utilisée selon la revendication 1, comprenant l'exosome à une concentration de 4 x 10⁸/ml ou plus ou de 33 ng/ml ou plus, de préférence de 4,7 x 10¹¹/ml ou plus, de manière plus préférable de 7,8 x 10¹²/ml ou plus en ce qui concerne la quantité de protéines.

3. Composition destinée à être utilisée selon la revendication 1 ou 2, le cancer étant un cancer colorectal, un cancer de la prostate ou un cancer du sein.

4. Composition destinée à être utilisée dans un procédé de traitement d'une infection virale ou bactérienne, la composition comprenant un exosome en tant que principe actif, l'exosome étant extrait de levures contenues dans du moût de levure de saké, du miso vieilli un an ou plus ou du miso vieilli trois ans ou plus.

5. Procédé cosmétique pour la prévention de la perte d'élasticité des tissus ou de l'amélioration de l'élasticité des tissus, ledit procédé comprenant l'administration d'une composition comprenant un exosome en tant que principe actif, l'exosome étant extrait de levures contenues dans du moût de levure de saké, du miso vieilli un an ou plus ou du miso vieilli trois ans ou plus.

6. Procédé cosmétique selon la revendication 5, dans lequel le tissu est la peau.

7. Composition destinée à être utilisée selon l'une quelconque des revendications 1 à 4 ou procédé cosmétique selon la revendication 5 ou 6, l'exosome étant produit par un procédé comprenant :
(a) la centrifugation du moût de levure ou du moromi de saké ou du miso vieilli un an ou plus, ou du miso vieilli trois ans ou plus à 10 000 à 20 000 g
(b) l'ultracentrifugation du surnageant obtenu à l'étape (a) à 10 000 à 210 000 g
(c) la collecte d'un précipité en tant que fraction exosomale.
